# EUROPEAN PATENT APPLICATION

(11) **EP 4 134 073 A1**
(43) Date of publication of application: **15.02.2023**
(21) Application number: 21190888.4
(22) Date of filing: 11.08.2021
(51) Int. Cl.: A61K 9/51, A61K 31/05, A61K 31/352, A61P 25/04

(54) **PHARMACEUTICAL COMPOSITIONS COMPRISING DENDRITIC NANOCARRIERS AND CANNABIS ACTIVE AGENTS**

(71) Applicant: HM HerbaMedica GmbH, 12435 Berlin (DE); DendroPharm GmbH, 12107 Berlin (DE)
(72) Inventor: Zierau, Klaas, 12435 Berlin (DE); Höhne, David, 12435 Berlin (DE); Moré, Sam Dylan, 12107 Berlin (DE)
(74) Representative: Moré, Solveig Helga

(57) **Abstract**

The present invention relates to formulations for administration of cannabis active agents, preferably, cannabis extracts. It provides a composition or kit comprising a plurality of cannabis active agents and a hyperbranched dendritic core-multishell-nanocarrier or core-shell nanocarrier, such as a hyperbranched dendritic polyglycerol nanocarrier, as well as corresponding pharmaceutical compositions and methods of preparing the same. Preferably, the cannabis active agents, e.g., THC and CBD, are stabilized by the nanocarrier, and their transdermal or transmucosal bioavailability is enhanced. The pharmaceutical compositions can be for use in treating a condition as a stand-alone pharmaceutical or an add-on pharmaceutical selected from the group comprising chronic pain, especially neuropathic pain, chronic inflammation, rheumatoid arthritis, diseases that are an expression of endocannabinoid deficiency, migraine, fibromyalgia, irritable bowel syndrome, Crohn's disease and ulcerative colitis, multiple sclerosis; nausea, vomiting, anxiety, Gilles-de-la-Tourette syndrome, Parkinson's disease, attention deficit disorder (ADD) post-traumatic stress disorder (PTSD) and sleep disorders, or as a potential antineoplastic therapeutic, as an add-on antiepileptic drug or for appetite stimulation.

## Description

The present invention relates to formulations for administration of cannabis active agents, preferably, cannabis extracts. It provides a composition or kit comprising a plurality of cannabis active agents and a hyperbranched dendritic core-multishell-nanocarrier or core-shell nanocarrier, such as a hyperbranched dendritic polyglycerol nanocarrier, as well as corresponding pharmaceutical compositions and methods of preparing the same. Preferably, the cannabis active agents, e.g., THC and CBD, are stabilized by the nanocarrier, and their transdermal or transmucosal bioavailability is enhanced. The pharmaceutical compositions can be for use in treating a condition as a stand-alone pharmaceutical or an add-on pharmaceutical selected from the group comprising chronic pain, especially neuropathic pain, chronic inflammation, rheumatoid arthritis, diseases that are an expression of endocannabinoid deficiency, migraine, fibromyalgia, irritable bowel syndrome, Crohn's disease and ulcerative colitis, multiple sclerosis; nausea, vomiting, anxiety, Gilles-de-la-Tourette syndrome, Parkinson's disease, attention deficit disorder (ADD) post-traumatic stress disorder (PTSD) and sleep disorders, or as a potential antineoplastic therapeutic, as an add-on antiepileptic drug or for appetite stimulation.

Cannabis is a genus of flowering plant in the family *Cannabaceae,* e.g., *Cannabis sativa, Cannabis indica* and *Cannabis ruderalis.* The plant is also referred to as hemp. Marijuana or cannabis are also names for a psychoactive drug from the cannabis plant. Herein, cannabis refers to plants or plant parts of *Cannabaceae.* Cannabis active agents, e.g., phytocannabinoids and terpenes have a high number of biological and pharmaceutical activities. However, their practical use is often impeded by disadvantages in handling and storage. They are prone to chemical conversion and degradation, e.g., by oxidation, isomerisation, polymerisation, or rearrangements, mainly depending on environmental parameters such as temperature, light and oxygen. These stability issues can lead to a reduction of efficacy. Furthermore, cannabis active agents have disadvantageous physico-chemical characteristics, such as low water solubility and high volatility that can render handling and use impractical.

Cannabinoid-comprising extracts can be prepared with a multitude of methods, e.g., with sub- or supercritical CO₂ (e.g., WO 2004016277 A2) hydrocarbons, alcohols, or by heating/pressure, and they can be purified by winterization (precipitation) in alcohols, distillation or chromatographic separation. The underlying techniques are well established and are extensively used in the field of phytopharmacology.

Conventional extraction and separation methods utilize polar and/or unpolar solvents and those techniques have the disadvantage to be expensive, and they need a high level of maintenance. Further, by using solvents, there is a high possibility of extracting undesirable components from the plant matter.

However, when extracting active constituents from glandular hairs of resin-producing plants, such as cannabis, while all methods may be used, there is no need to use complex extraction procedures. Since the pharmacologically relevant active ingredients (cannabinoids, terpenes) are found in the highest concentrations in the glandular hairs (trichomes) of the female flower of the cannabis plant, a purely mechanical separation process can be used, thus eliminating the need for solvents or heat and pressure. This method, known as "solventless separation", uses a physical force to separate the trichomes from the plant matter. Thermolabile compounds, such as the volatile terpenes, can be protected and no potentially toxic residues from the extraction process remain in the extract. For example, a process as disclosed in US 9,718,065 B1 can be used, which is not technologically demanding and requires little maintenance.

Cannabinoids are highly lipophilic, with LogP values of 5-9, i.e., they are practically insoluble in water. These are characteristics that prevent efficient absorption via oral administration, such as drops, sprays or tablets. Due to these characteristics, the cannabinoids are not suitable for traditional pharmaceutical administration methods, such as oral application, which is why smoking/vaporisation of the whole flower still is a preferred administration method. Therefore, it is necessary to use formulations that improve the absorption of such highly lipophilic components. Various processes and technologies have been developed and used in modern formulations that lead to better absorption of such highly lipophilic substances, e.g., as disclosed in US7025992B2, ES2444641T3 , US9718065 B1 or US10080736B2.

Nano-CBD or cannabis nanoemulsions are oily cannabis extracts or cannabis isolates dissolved in oil that are changed in their physico-chemical characteristics by a mechanical process with the aid of solvents and stabilizing agents. Thus, in general, micro- or nanoemulsions are formed under the influence of energy, e.g., ultrasound, so that the cannabinoid droplets are finely dispersed in a water-based solvent. Due to their small particle size, the lipophilic active agents in such emulsions have improved penetration characteristics through epithelia. However, the preparations obtained in this way are subject to strong degradation reactions, such as oxidative stress, and thus have a reduced stability.

Another technology is based on so-called SEDDS (Self-Emulsifying Drug Delivery Systems). Cannabinoids dissolved in terpenes or triglycerid oil or a mixture thereof form a self-emulsifying composition, applied to an adsorbing powder, which thus form a solid dosage form that is self-emulsifying when contacted with body liquids, which leads to a very fine sub-micron emulsion with a plurality of particles having a medium particle size below a micron, and which thus have a significantly enhanced oral bioavailability of at least 50%. The addition of excipients to this end is however controversial under toxicological aspects. The pharmacokinetic characteristics of state of the art oral cannabis medicaments, such as oily extracts, but also sprays for oro-mucosal application using ethanol as a solvent (Nabiximols (Sativex^{®}), GW Pharmaceuticals) all show a low bioavailability of about 6-10% (Ohlsson et al., 1980. Clin Pharmacol Ther 28 (3): 409-16; Constantin et al., 2019. Expert Opinion on Drug Delivery, DOI: 10.1080/17425247.2019.1653852; Karschner et al., 2011. Clin Chem. 57(1):66-75). Furthermore, the ingredients of the spray do not stick to the mucosa and thus in large parts arrive in the stomach. The resorption in the gastrointestinal tract is largely dependent on the filling of the stomach, and is thus subject to strong individual fluctuations.

Phytocannabinoids formulated for oral application have low solubility and do not pass well through the epithelial barrier, and can thus only be taken up by the patients in low amounts.

In light of this, the present inventors addressed the problem of providing a formulation of cannabis active agents that overcomes some or all of these problems, and which advantageously has an improved bioavailability and an improved stability.

This problem is solved by the subject-matter of the claims. In particular, the present invention provides a composition or kit comprising
a) a hydrophilic nanocarrier comprising a hyperbranched dendritic polymer selected from the group comprising a core-multishell-nanocarrier and a core-shell nanocarrier, and
b) at least one, preferably, a plurality of cannabis active agents, preferably, a cannabis extract, and,
c) optionally, a carrier solution.

Preferably, the components a) and b) are combined into a composition of the invention.

The inventors have surprisingly found that transdermal and transmucosal administration of a composition of the invention leads to an increased bioavailability compared to conventional transmucosal administration, e.g., of Sativex^{®}. The bioavailability of the active agents, in particular, THC after oromucosal administration of Sativex^{®} is 4-10% (w/w). After oromucosal administration of the composition of the present invention, bioavailability of THC is at least 15%, preferably, 20%-100%, 25%-90%, 30-80% or 40-70%.

Bioavailability is the fraction (%) of an administered drug that reaches the systemic circulation.

By definition, when a medication is administered intravenously, its bioavailability is 100%. However, when a medication is administered via routes other than intravenous, its bioavailability is generally lower due to intestinal endothelium absorption, enzymatic degradation and first-pass metabolism. Thereby, mathematically, bioavailability equals the ratio of comparing the area under the plasma drug concentration curve versus time (AUC) for the extravascular formulation to the AUC for the intravascular formulation. AUC is utilized because AUC is proportional to the dose that has entered the systemic circulation (Wikipedia). Bioavailability can be determined e.g., as taught by Karschneret al., 2011. Clin Chem. 57(1):66-75.

In one embodiment, a) and b), and optionally, c), are part of a kit, which can be mixed together before administration. This also leads to increases in bioavailability. However, a composition comprising a) and b), i.e., wherein, a) and b) are in one composition, may additionally increase storage stability, and is therefore preferred.

A kit, which as preferred, may comprise a) and b) in one composition, or which may comprise them separately, may optionally further comprise a carrier solution. A carrier solution mainly comprises a buffer and/or solvent and may be used to adjust the concentration of active ingredients. The desired concentration setting depend on the individual dose finding and may be different for each patient. A kit, in particular a kit comprising a carrier solution, preferably is a product for medicinal use, wherein the composition comprising the active agents may be diluted, e.g., in a pharmacy.

### Cannabis active agents

The cannabis active agent can be a phytocannabinoid or terpene derived or derivable from a cannabis plant, an analogue or derivative thereof, or a synthetic or semi-synthetic cannabinoid. A cannabinoid is capable of binding and activating cannabinoid receptor CB₁ and/or CB₂. CB₁ receptors are found primarily in the brain, more specifically in the basal ganglia and in the limbic system, including the hippocampus and the striatum. They are also found in the cerebellum and in both male and female reproductive systems. CB₁ receptors are absent in the medulla oblongata, the part of the brain stem responsible for respiratory and cardiovascular functions. CB₁ is also found in the human anterior eye and retina, and on peripheral nerves (Wikipedia). CB₂ receptors are predominantly found in the immune system, or immune-derived cells, with varying expression patterns. They are further found in the peripheral nervous system, but also expressed by a subpopulation of microglia in the human cerebellum. CB₂ receptors appear to be responsible for immunomodulatory and possibly other therapeutic effects of cannabinoid as seen *in vitro* and in animal models (Wikipedia).

Cannabinoids can belong to the classes of the cannabigerol type, the cannabichrome type, the cannabidiol type, the tetrahydrocannabinol- and cannabinol type, the cannabielsoin.type, the iso-tetrahydrocannabinol type, the cannabicyclol type or the cannabicitran type.

Exemplary cannabinoids with proven pharmacological properties are THC (tetrahydrocannabinol), THCA (tetrahydrocannabinolic acid), CBD (cannabidiol), CBN (Cannabinol), CBDA (cannabidiolic acid), CBG (cannabigerol), CBC (cannabichromene), THCV (tetrahydrocannabivarin), THCP (tetrahydrocannabiphorol) and CBDV (cannabidivarin), or can be synthetic cannabinoids: e.g. Nabilone (synthetic THC), biosynthetic cannabigerol, biosynthetic cannabidiol, or synthetic THCV. Advantageously, as it has been shown that undesired effects of certain cannabis active agents, e.g., THC, may be reduced in the presence of further cannabis active agents, the composition of the invention comprises a plurality of cannabis active agents. The inventors could show that an association with the nanocarrier in the context of the invention can be obtained for a plurality of such active agents, and the profile of physiologic effects is improved if a plurality of such agents are administered in combination with the nanocarrier. In particular, advantageously, bioavailability and/or stability of several such agents, e.g., THC and CBD is improved in parallel.

Preferably, tetrahydrocannabinol (THC) is a cannabis active agent comprised in the composition or kit of the invention. THC is one of at least 113 cannabinoids identified in cannabis, in particular, the principal psychoactive constituent of cannabis. There are multiple isomers, but, throughout the invention, THC preferably is the delta-9-THC isomer with the chemical name (-)-*trans*-Δ⁹-tetrahydrocannabinol (Δ⁹-THC). THC is an agonist of at least CB₁ and CB₂ receptors. It further has antagonistic effects on 5-HT3 receptors involved in emesis. THC also has an effect on capsaicin-sensitive perivascular neurons. It has an antioxidative effect and is neuroprotective. Δ⁸-Tetrahydrocannabinol (Δ⁸-THC) is psychoactive, but less potent than Δ⁹-THC. THC can also be a combination of THC isomers, e.g., a combination of Δ⁹-THC and Δ⁸-THC.

Another preferred cannabis active agent comprised in the composition or kit of the invention is CBD (cannabidiol). CBD is non-psychotropic. Recent evidence shows that the compound counteracts cognitive impairment associated with the use of cannabis. Cannabidiol has little affinity for CB₁ and CB₂ receptors, but acts as an indirect antagonist of cannabinoid agonists. It was found to be an antagonist at the putative new cannabinoid receptor, GPR55, a GPCR expressed in the caudate nucleus and putamen. Cannabidiol has also been shown to act as a 5-HT1A receptor agonist. CBD can interfere with the uptake of adenosine, which plays an important role in biochemical processes, such as energy transfer. It may play a role in promoting sleep and suppressing arousal.

CBD shares a precursor with THC and is the main cannabinoid in CBD-dominant Cannabis strains. CBD has been shown to play a role in preventing the short-term memory loss associated with THC. There also is tentative evidence that CBD has an anti-psychotic effect (Wikipedia).

A plurality of active agents may be two active agents. The composition may also comprise at least three, at least four, at least 5, at least 6, at least 7, at least 8, at least 9, at least 10, at least 20, at least 30 or at least 50 cannabis active agents. In a preferred embodiment, the composition or kit comprises a cannabis extract comprising at least 10, at least 20, at least 30 or at least 50 cannabis active agents. It is possible to add one or more specific active agents, e.g., a synthetic cannabinoid and/or terpene, to an extract comprising cannabis active agents.

Advantageously, in a preferred embodiment, the composition or kit of the invention comprises both THC and CBD, typically, in a defined ratio. For example, the ratio of THC to CBD can be 1:100 to 100:1, e.g., 1:50 to 50:1, 1:30 to 30:1 or 1:10 to 10:1, or, e.g., about 1:1. Other ratios may be used.

Ratio refers to the molar ratio, which is about the same as the mass ratio. (THC= 314,45 g/mol, CBD 314,47 g/mol). Cannabinoids, including their quantity, can be reliably analysed, e.g., by HPLC-MS. About refers to +/- 10%. Different ratios of THC to CBD can be desired for different purposes, e.g., studies suggest that CBD may attenuate some effects of THC such as anxiety, cognitive deficits, and psychosis (Boggs et al., 2018. Neuropsychopharmacology 43(1); Colizzi et al., 2017.

For standardized medication, a defined, stable ratio of THC to CBD is desired. It has been shown that such as stable ratio can be advantageously provided by the composition of the present invention, as the nanocarrier stabilizes both cannabinoids, and prevents their degradation and conversion. In conventional compositions, such degradation and conversion processes take place at different rates. Accordingly, depending on the environment, THC can be degraded more quickly than CBD or vice versa. Thus, in conventional compositions, the ratio of THC to CBD (and to other cannabis active agents) changes over time. This effect is much reduced or, preferably, absent, in the composition of the invention.

Therefore, preferably, in the composition of the invention, the concentration of cannabis active agents, in particular, of THC and/or CBD, is stable (i.e., within the range of 90-110%) for at least 6 months, preferably, for at least 12 months. It can also be stable for at least 18 months, most preferably, for at least 24 months.

Further, advantageously, in the composition of the invention, the ratio of THC to CBD is stable (90-110%) for at least 6 months, preferably, for at least 12 months. It can also be stable for at least 18 months, most preferably, for at least 24 months.

Preferably, the composition or kit of the invention comprises further cannabis active agents in addition to THC and CBD, e.g., further cannabinoids and/or terpenes. The cannabis active agents in the composition may be naturally derived, e.g., from a cannabis extract, synthesized cannabinoids derivable from cannabis, synthetic cannabinoids or a mixture thereof.

Cannabinol (CBN) is a mildly psychoactive cannabinoid found only in trace amounts in *Cannabis,* and is mostly found in aged *Cannabis.* Pharmacologically relevant quantities are formed as a metabolite of THC. CBN acts as a partial agonist at the CB₁ receptors, but has a higher affinity to CB₂ receptors; however, it has lower affinities relative to THC. There is increasing interest for using CBN as a therapeutic agent in glaucoma or dermatological diseases (e.g., epidermolysis bullosa).

CBG (cannabigerol) is a non-psychoactive cannabinoid. It antagonizes CB₁ receptors, and is both an alpha2-adrenoceptor agonist and moderate 5HT1A receptor antagonist. Cannabigerol displays CB₁ and CB₂ binding affinity, and has been evaluated in laboratory models of colitis.

Phytocannabinoids are known to occur in several plant species besides cannabis. These include *Echinacea purpurea, Echinacea angustifolia, Acmella oleracea, Helichrysum umbraculigerum,* and *Radula marginata.* The best-known cannabinoids that are not derived from Cannabis are the lipophilic alkamides (alkylamides) derivable from *Echinacea* species, most notably the cis/trans isomers dodeca-2E,4E,8Z,10E/Z-tetraenoic-acid-isobutylamide. Some of these alkylamides have shown affinities to the CB₂-receptor.

In a preferred embodiment, the composition or kit of the invention does not only comprise at least one, preferably, a plurality of cannabinoids (e.g., at least THC and CBD), but also comprises at least one terpene derivable from cannabis, preferably, a plurality of such terpenes. Cannabis active agents that belong to the class of terpenes are, e.g., the monoterpene β-myrcene and the sesquiterpenes β-caryophyllene and α-humulene. These occur most frequently and with the highest concentrations. However, α-pinene, limonene, and linalool, as well as the sesquiterpene bisabolol and (E)-β-farnesene, also occur with decreasing frequency.

For example, beta-caryophyllene, a component from the essential oil of cannabis has also been identified as a selective agonist of peripheral CB₂-receptors, *in vivo.* It thus acts directly on the endocannabinoid system.

Terpenes are highly active pharmacological substances and are effective at very low concentrations (in a single-digit nanomolar range).

As lipophilic substances that can easily penetrate cell membranes, they interact with various structures, such as ion channels, neurotransmitter receptors, G-protein-coupled receptors, and enzymes.

There is evidence for potential modulation of cannabis effects by terpenes, namely, some undesirable effects of THC could be attenuated by terpenes, such as the negative effect of THC on short-term memory. This approach of high interest in treatment of Alzheimer's disease.

Furthermore, THC is known for negative psychomimetic effects, such as depersonalization and anxiety. These effects, in turn, may be mitigated by the antidepressant, sedative, and anxiolytic effects of terpenes.

The analgesic effects of cannabis are largely attributed to cannabinoid action, but β-myrcene also exerts both potent analgesic and anti-inflammatory effects. Other terpenes, such as cavacrol, even exert significantly stronger anti-inflammatory effects than THC alone.

Due to the preferred production or extraction process of the cannabis extracts used in the composition of the invention, detailed below, the content of terpenes is high, e.g., at least 0.05 % v/w, e.g., 0.05-10% (v/w), 1-10% (v/w) or 3.5-5 % (v/w).

In a preferred embodiment, the plurality of cannabis active agents are in a cannabis extract. The extract typically is a cannabis extract from one variety of cannabis plants. It may also be a mixture of two or more varieties (chemovars), which can be helpful to obtain a specific ratio of THC:CBD, in particular, if the desired ratio is not naturally, or not reliably present in one chemovar. Preferably, a cannabis extract comprises at least cannabinoids such as THC and CBD. It may further comprise terpenes extracted from cannabis.

The invention also provides a method for preparing a composition or kit of the invention, comprising preparing a cannabis extract and providing a nanocarrier, as defined herein, wherein, for preparing a composition of the invention, the cannabis extract and the nanocarrier are mixed. Optionally, two or more cannabis extracts are mixed with the nanocarrier. Optionally, the method further comprises covalently linking least one of the cannabis active agents, e.g., THC, or all cannabis active agents in the extract with the nanocarrier. If desired, the product of this linking reaction can, e.g., after washing, be mixed with a further cannabis extract or specific active agents, e.g., a CBD-comprising extract not comprising significant amounts of THC, or with isolated CBD.

Plant material from which such extracts are preferably derived may e.g., be cannabis flos. Specifically, trichomes from cannabis plants are preferably extracted. The plant material advantageously is from cannabis chemovars with high medicinal-therapeutic value.

Recent classification models attempt to form groups, so-called chemovars, by analyzing percentage distributions of terpenes and cannabinoids, which are characterized by clearly distinguishable chemoprofiles. In this way, the diverse strains of the cannabis industry could be divided into groups with chemical degrees of relationship, and thus with distinguishable pharmacological effects.

Different preparation processes for cannabis extracts or for isolating active agents from cannabis plant material are known in the art. It is possible to use any of these, e.g., sub- or supercritical CO₂, hydrocarbons, alcohols, or heating/pressure, and/or they can be purified by winterization (precipitation) in alcohols, distillation and/or chromatographic separation, e.g. as described above. Preferably, however, the cannabis active agents are prepared in a solvent-free extraction process. Such processes are traditionally known in the art, and preferred processes are described below.

Preferably, the method for preparing the cannabis active agents comprises separating, purifying and activating the plant active ingredients in the resin of glandular trichomes of cannabis plants, e.g., of one chemovar, in a nonsolvent-based and therefore nontoxic traditional extraction process. The resulting cannabis active agents, and, in particular, the composition of the invention can be standardised, e.g., with regard to their THC: CBD profile and/or THC content. The standardized composition may, e.g., mirror the unique phytocannabinoid / terpene profile of the donor plant. Due to the nanocarrier, it however shows improved pharmacokinetics.

For example, a process for preparing cannabis active agents may comprise
1. Harvesting the fresh plant and isolation of florescences.
2. Freezing.
3. Breaking up frozen florescences
4. Ice water filtration for isolation of trichomes.
5. Collection of trichomes
6. Cavitation by ultrasound and winterisation in ethanol.
7. Separation of volatile components (e.g., terpenes) from phytocannabinoids and other resin components by distillation.
8. Decarboxylation of inactive forms of phytocannabinoids with heat (leading to an oil extract).
9. Optionally, fusion of the oil extract and the terpene distillate.
10. Optionally, standardization of the oil extracts or the product of step 9
11. Encapsulation in the nanocarrier (non-covalent encapsulation by mixing or covalent encapsulation by linkage through esterification).

Preferably, both step 9 and step 10 are carried out. Additional cannabis active agents - or other active agents - may also be added. Typically, the steps are carried out in the specified order, but steps 9 and/or 10 may also be carried out after step 11. The formed composition thus preferably comprises cannabinoids, e.g., THC and CBD and terpenes and the nanocarrier.

### Nanocarriers

The composition or the kit of the present invention comprises a hydrophilic nanocarrier comprising a hyperbranched dendritic polymer selected from the group comprising a core-multishell-nanocarrier and a core-shell nanocarrier. The nanocarrier is an amphiphilic unimolecular nanocarrier of dendritic structure, and it comprises a dendritic core and at least one shell, wherein an inner shell is coupled to the dendritic core via a first linker (a core-shell-nanocarrier), wherein, in the case of a core-multishell-nanocarier, further, an outer shell is coupled to the inner shell via a second linker. While a core-shell nanocarrier with one shell may be used, preferably, the composition or kit of any of the invention comprises a nanocarrier consisting of a dendritic core and at least two shells, wherein an inner shell is associated with the dendritic core via a linker, and an outer shell is associated with the inner shell with a linker. In this context, layers of the nanocarrier which differ in their hydrophilicity or hydrophobicity are referred to as core or shell. For example, the core may be hydrophobic and the shell hydrophilic. One possible construction for a core multi-shell nanocarrier is, e.g., core - ester bond - diacid - ester bond - mPEG. Alternatively, the construction may be core - ester bond - diacid - ether bond - PEG.

The nanocarrier may be a hyperbranched dendritic polyglycerol polymer, a hyperbranched dendritic polylactate polymer or a hyperbranched dendritic polyglycerol-polylactate polymer. Preferably, it is a hyperbranched dendritic polyglycerol polymer.

The shell may alternatively, e.g., comprise poly-2-alkyl-2-oxazolines. The polarity can be selected by choice of an appropriate alky. For example, if the alkyl is methyl or ethyl, the shell is more polar than if the alkyl is longer, e.g., n-hexyl, n-heptyl, or n-octyl. This shell can be linked to a hyperbranched dendritic core, e.g., a functionalized polyglycerol core, for example with an ester that can be generated using succinic anhydride.

Still further alternative nanocarriers comprise Poly(3-Ethyl-3-hydroxy-methyloexetan (PEHO) alkyl chains linked with imidazolium. Nanocarriers with hyperbranched PEHO cores and at least one thermoresponsive shell comprising poly-2-alkyl-2-oxazolines may be used. Other options for nanocarriers are POx based brush-like PIL nanocarriers or amphiphilic di-block polymers of polyisobutylen (PIB) having a chain length of 18-25, hydrophilic polyethyloxazolin (PEtOx) having a chain length of 8-12 , which may form gels or having a chain length of 20-40, which may form micelles.

The nanocarriers used in the invention may be hydrophilic. This may be due to the presence of polar, that is to say hydrophilic and/or charged groups, e.g. hydroxyl groups, carboxyl groups, amino groups, sulfate groups or succinate groups at the surface. They may be anionic or cationic functional groups, i.e., the nanocarrier optionally is a negatively or positively charged nanocarrier, preferably, a negatively charged nanocarrier.

The functional groups, and the nanocarrier, may be biodegradable (e.g., Wallert et al., 2021. Macromol. Mater. Eng. 2000688). The nanocarrier may thus be degraded into a core and the components of the shell, wherein both are non-toxic and can be eliminated from the body due to their small size. Hydrophilic nanocarrier comprising a hyperbranched dendritic polyglycerol polymer disclosed in Wallert et al., 2021 may be used in the present invention.

In the nanocarriers of the invention, optionally, 2-50%, e.g. 5-15%, of the functional terminal groups are functionalized with ionic groups, such as with negative charge. These groups can also be inside the nanocarrier.

Hyperbranched polymers may be dendritic polymers or dendrimers. Dendrimers are chemical compounds whose structure is perfectly branched from a branching core like a tree. This is called dendrimer, if these branches consist of repetitive units, thus giving a radial symmetry. These must therefore - if one starts from a core - contain branches, otherwise one would receive a chain. There can be a branching into two or more links.

It is easier to produce dendritic or highly branched polymers (hyperbranched polymers), with very similar properties, in which not all possible branching sites are branched. In the context of the invention, the nanocarriers are preferably based on a hyperbranched polyglycerol (hPG).

In one embodiment, the nanocarriers are neutral or anionic. Corresponding nanocarriers are e.g. disclosed in WO 2006/018295 A2. Nanocarriers disclosed in Radowski et al (Angew Chem Int Ed 2007 46, 1265-1269); Fleige et al. (Macromolecules 2012, 45, 9452-9459), Fleige et al. (Nanocarriers, Vol. 1, 2013, 1-9, WO2011/095311), or Haag et al. (Macromolecules, 2000, 33, 8158-8166) can also be used in the context of the invention. A unimolecular sulfated polyanionic nanocarrier, especially a unimolecular polyanionic polyglycerol micelle with a hydophilic shell and a hydrophobic core (EP application no. 14 161 579.9) can also be used. Nanocarriers, which, like those nanocarriers, preferentially accumulate in inflamed areas after systemic administration are well-suited for use in the context of the present invention.

Preferred nanocarriers are disclosed in WO 2015/172769 A2. The nanocarrier may be configured such that
a) the dendritic core of the nanocarrier is made of polyglycerol, preferably with a molecular weight of 3-20 kDa, more preferably 7-10 or 8-9 kDa; and/or
b) the inner shell of the nanocarrier is a preferably linear alkyl chain with a carbon length of C2 to C40, e.g., C8, C9, C10, C11, C12, C13, C14, C15, C16, C17 or C18, preferably, C10-C18, such as or C12-C15; and/or
c) the outer shell is polyethylene glycol having the structural formula (-CH₂-CH₂O-)ₙ, with n=3-130, which bears a terminal methyl group, a hydroxyl group or a carboxyl group, preferably a methyl group and/or
d) the first linker is an ester or amide linkage; preferably, an amide linkage, and/or
e) the second linker is an ester bond,
wherein preferably all features of a-e apply.

In a preferred embodiment, the nanocarrier is DendroSol^{®} available from Dendropharm, Berlin, DE.

The nanocarriers may comprise dendritic polyglyceroldodecanic acid polyethylene glycolate. It can be characterized by way of example as follows:
Nomenclature formula: hPG₁₀₀₀₀(-NH₂)_{0.7}(C₁₂mPEG₃₅₀)_{1.0} (Mn=350)
Alternative nomenclature: hPG₁₀₀₀₀(-NH₂)_{0.7}(C₁₂mPEG₆)_{1.0} (6 repeating units on average)

hPG10k has approximately 135 functional groups, approximately 40-80% of them, for example approximately 70% having reacted to amines (index number 0.7).

In total, i.e., including the core and shell or shells, the nanocarrier may have e.g., a molecular weight of 100 g/mol-100,000 g/mol.

In one embodiment, molecular weights of 100-10,000 g/mol, or 200-990 g/mol, such as 300-600 g/mol, are preferred. Such sizes may be particularly advantageous for transdermal, e.g, transmucosal administration.

The nanocarrier can aggregate and can absorb guest molecules (e.g., the active agents) during this aggregation. Unimers and aggregates are in an equilibrium, which shifts with increasing dilution in direction of unimers, but with slow kinetics. In addition, especially depending on polarity and pH of the environment, a release of the guest molecules from the aggregates takes place. Therefore, the nanocarrier does not only act as emulsifier but also as a permeation modulator, improving, on the one hand, the kinetics of drug release, and, on the other hand, helping to overcome the dermal barrier. Due to an interaction with the stratum corneum, an optimal insertion into upper layers of skin with subsequent favourable release profile can be achieved.

It has been shown that nanocarriers as used in the invention shuffle the active agent over the epithelial barrier, e.g., in oro-mucosal administration, but that they remain in the stratum corneum (Jager et al., 2018. Characterization of hyperbranched core-multishell nanocarriers as an innovative drug delivery system for the application at the oral mucosa. J. Periodontal Res. 53(1):57-65; Dommisch et al., 2021. Characterization of an ester-based core-multishell (CMS) nanocarrier for the topical application at the oral mucosa. Clin Oral Investigation. doi: 10.1007/s00784-021-03884-x). Preferably, with nanocarriers of these sizes, the active agents are not covalently attached.

In the composition or kit of the invention, the nanocarrier may alternatively have a molecular weight of 1,000 g/mol-100,000 g/mol, e.g., 5,000-75,000 g/mol, 8,000-50,000 g/mol or 30-40,000 g/mol, preferably, 3,000-10,000 g/mol. Typically, the nanocarrier is a mixture of nanocarriers of different weights, as derived from the production process, so, typically, the mean molecular weight is decisive. Molecular weight distributions (Mn, Mw) and the dispersity can be determined by GPC as taught by Wallert et al., 2021.

The diameter of the nanocarrier may be 5-40 nm, e.g., 8-22 nm, or 10-20 nm. The size may be determined by dynamic light scattering, e.g., as described by Fleige at al., 2014. J. Control. Release 185:99-108. Such nanocarriers are preferred for systemic, e.g., intravenous administration, intraperitoneal, intraarticular, subcutaneously or perioperatively. It has been found that such nanocarriers efficiently prevent transfer of systemically administered associated active agents, e.g., analgesics, to the CNS (WO 2017/097291 A1). The active agents may be covalently or non-covalently associated. Intramuscular administration is also possible for delayed release, in particular for smaller sized nanocarriers covalently linked to the active agents.

In the composition of the present invention, preferably, at least one of the cannabis active agents, e.g., THC, has a peripheral analgesic effect and a central psychoactive effect. If the size of the nanocarrier is chosen to be in the range of 1,000-100,000 g/mol or, e.g., 5-40 nm, as described herein, in particular, if the active agent having a central psychoactive effect, e.g., THC is covalently linked to the nanocarrier, the central effect of such active agents is blocked. Accordingly, the undesired psychoactive effects, when the composition is e.g., used for its analgesic properties, are reduced or, preferably, avoided, e.g., compared to a composition leading to the same plasma levels of THC after administration that does not comprise said nanocarriers.

In general, in particular, for use in transdermal and transmucosal administration, the cannabis active agents in the composition of the invention are non-covalently associated with the nanocarrier. Non-covalent associations are selected from the group comprising hydrophobic interactions, Van der Waals-interactions, or interactions of aromatic pielectrons.

Alternatively, in particular, if the composition is for use in intravenous administration, at least one active agent, preferably, THC, is covalently associated with the nanocarrier. This can be particularly helpful if a predominantly peripheral effect, e.g., of THC, is desired. Optionally, a plurality of cannabis active agents are covalently associated with the nanocarrier. Cannabinoids such as THC have a phenolic hydroxyl group and can thus be linked to the nanocarrier by esterification. An acid can be used as a linker, e.g., a C2-C20 carbonic acid, and the anhydride of said acid may be used for the reaction, or a carboxylic group of the nanocarrier may be used for forming an ester bond with the hydroxyl group of the cannabinoid. Acid, e.g., acetate derivatives of THC are known to be cleavable in vivo, as they serve as prodrugs of THC.

It is also possible that different cannabis active agents are partly covalently and partly non-covalently associated with the nanocarrier. For example, at least one active agent, preferably, CBD, may be non-covalently associated with the nanocarrier, wherein, at least one active agent, preferably, THC is covalently associated with the nanocarrier. Such mixtures can be prepared, e.g., using cannabis extracts from different chemovars comprising a) substantially THC, but not CBD, or b) CBD, but not THC. An extract comprising THC, but no substantial amounts of CBD can thus be prepared and the cannabinoid covalently linked to the nanocarrier. Then, e.g., after appropriate washing, CBD or an extract comprising CBD, but substantially no THC can be added for non-covalent association. With appropriate selection of the nanocarrier, as disclosed herein, this would reduce or prevent central effects of THC, but not of CBD. Alternatively to extracts, isolated active agents THC and CBD may be used.

In a preferred embodiment, an inventive composition comprises
a) 0.5-100 mg/mL of the nanocarrier, preferably about 2.5-50 mg/mL, about 5-35 mg/mL (w/w) or about 5-10 mg/mL,
b) 0.025-40 mg/mL of cannabis active agents, e.g., 0.1-20 mg/mL, 0.5-10 mg/mL or 1-3 mg/mL of cannabis active agents, wherein, preferably, the cannabis active agents considered for this calculation are THC and/or CBD, wherein the composition may optionally comprise THC and CBD in a ratio disclosed herein. Other cannabis active agents may also be comprised.

The composition may include further excipients, e.g., solvents, stabilizers or emulsifiers, and/or active substances, for example dexpanthenol.

The solvent for the composition of the invention for transmucosal administration typically is water. The composition can either be a solution or an emulsion. An oil-in-water emulsion can be used.

Another exemplary solvent is oil, e.g., at least one fatty oil selected from the group of plant fatty oils comprising peanut oil, almond oil, sunflower oil, linseed oil, olive oil or evening primrose oil, or a synthetic fatty oil such as mineral oil. Plant oils are preferred over mineral oil due to their lower allergic potential and better absorption in the context of the composition of the invention. In this case, hydrophobic nanocarriers are preferred. A water-in-oil emulsion can also be used.

The composition may also comprise ethanol, however, this is not necessary. The composition can thus be free of ethanol.

The composition may alternatively be formulated in saline (e.g., 0.9% NaCI) or buffer (e.g., phosphate-buffered saline, pH 7.2-7.4), e.g., for intravenous application, but also for transdermal or transmucosal application. It may be a sustained-release composition, e.g. in combination with hydrogels or lipogels suitable for long-term provision of the active agent.

The composition of the invention includes an amphiphilic nanocarrier acting as an emulsifier. The addition of an additional emulsifier is not therefore necessary. However, a further emulsifier may also be added. Such additional emulsifier may be cetylstearyl alcohol, propylene glycol or a combination thereof. PEG can also act as an additional emulsifier. Cetylstearyl alcohol is a mixture of cetyl alcohol (hexadecanol) and stearyl alcohol (octadecanol), which increases the stability of emulsions and can improve the texture of preparations.

The emulsifier preferably also acts as a penetration enhancer or tractor. Preferably, propylene glycol is avoided or substantially avoided, as this can lead to skin irritation, especially in larger concentrations.

In a preferred embodiment, the composition comprises at least one mucoadhesive excipient suitable for preventing or reducing the transfer of the composition of the invention to the stomach upon oromucosal administration, e.g., as disclosed in Grabovac et al. 2005. Adv Drug Deliv Rev. 57 (11): 1713-1723. For example, glycosaminoglycanes (GAG) are suitable to this end, e.g., hyaluronic acid, heparin or heparan sulfate, chondroitin sulfate or dermatanesulfate or keratan sulfate. Other suitable mucoadhesive excipients are polyacrylic acid, PAMAM (polymaleic acid, polyacrylic acid), PVMI MA or Helix aspergia muller glycoconjugate.

Further, the taste and/or mouthfeel of the composition can be improved, e.g., by addition of a flavouring agent. Flavouring agents such as mint, lemongrass, lemon, strawberry, ginger or cinnamon flavouring may thus be comprised in the composition of the invention. The composition may also comprise a sweetener, e.g. saccharose, xylitol, stevia extract and/or aspartam. Palatibility and mouthfeel may be improved, e.g., by addition of one or more hydrocolloids (Saha et al., 2010. J Food Sci Technol. 47(6): 587-597), e.g., starch, modified starch, xanthan, carboxymethyl cellulose, inulin, guar gum, locust bean gum, acacia gum, pectin and/or hydrochlorides.

In one embodiment, the composition according to the invention can be a gel or an ointment or a cream. It may be a water-in-oil suspension or an oil-in-water suspension. Gel, ointment or cream are semi-solid and spreadable. Preferably, a gel, ointment or cream is for transdermal application. A hydrogel can also be for transdermal or oromucosal administration.

The composition of the invention preferably is a spray, e.g., a spray for transdermal or, preferably, for transmucosal administration.

### Pharmaceutical compositions

The invention also provides a pharmaceutical composition or kit of the invention. It may be formulated for administration to a human. Alternatively, it may also be a veterinary pharmaceutical, e.g. formulated for administration to an animal, e.g., a pet, such as a cat, dog, horse, rabbit, guinea pig, hamster, mouse, rat or camel.

The pharmaceutical composition may be formulated for intravenous, intramuscular, intraperitoneal, subcutaneous, transdermal or transmucosal administration. Administration is also possible via an implant or a semi-automatic or manual pain pump system. While a local administration is possible, some benefits of the invention, namely, the suppression of the central action of the drug, are more apparent with systemic administration.

Preferably, the composition is administered transmucosally, transdermally or intravenously, most preferably, by transmucosal, in particular, by oro-mucosal application. Oro-mucosal administration may be buccal or sublingual.

Due to the combination with nanocarriers of the invention, several advantageous pharmacokinetic effects can be realized: Firstly, the increased bioavailability due to the encapsulation in the nanocarriers that permeate skin and mucosa more easily allows for efficient transdermal or mucosal administration. As oral application via the gastrointestinal tract is preferably avoided, the first-pass-effect is reduced. Thus, there are decreased total metabolization needs in the first place. The avoidance of the first pass effect also leads to a decreased formation of psychoactive metabolites such as 11-Hydroxy-Δ9-THC. Further, the potential of drug-drug interactions is decreased.

In case of a kit, components of the kit are preferably mixed before administration. A kit may also be a self-mixing or premixed two-component syringe. By mixing of the components of the kit, a composition of the invention is formed.

The composition or the components of the kit may be administered in combination with an analgesic selected from the group comprising an opioid or other analgetics, like a NSAR or NSAID or another anti-inflammatory agent, or another active agent active in the CNS, e.g., an antiepileptic or an antidementia agent. Administration in combination means that the agents may be administered together, e.g., after mixing, or separately within a short time span, e.g., within a day, within two hours, within an hour, within 30 min, or within 10 min. Administration may be with the same mode of administration, e.g., transdermally, or by different ways of administration. For example, the composition comprising nanocarriers and cannabis active agents may be administered transmucosally, and the opioid may be administered intravenously or orally. The opioid may be an opioid associated with a nanocarrier, as disclosed in WO 2017/097291 A1.

The pharmaceutical composition or kit of the invention may be for use in treating a condition selected from the group comprising pain, e.g., chronic pain or acute pain, neuropathic pain, inflammation-induced pain, pain associated with osteoarthritis, pain associated with cancer, seizures, e.g., seizures associated with Dravet syndrome, seizures associated with Lennox-Gastaut syndrome, or tuberous sclerosis complex or epilepsy, multiple sclerosis, spasticity, overactive bladder, increased intraocular pressure, emesis, insomnia, cancer, anorexia, cachexia, or for muscle relaxation, reducing gastric motility, and/or increasing appetite. For example, for treatment of pain, in particular, neuropathic pain, inflammation-induced pain, or pain associated with osteoarthritis a composition of the invention wherein THC is covalently associated with the nanocarriers may be used.

THC and CBD undergo two phases of metabolism in the liver. Phase I is conducted by the cytochrome P450 system. Phase II is glucuronidation of the phase I metabolites, facilitating fecal and urinary excretion by the kidneys.

Compared to conventional cannabis formulations, the composition of the present invention is particularly suitable for patient groups with reduced liver function, because less degradation products of the cannabinoids are administered with the highly stable product. Because of the higher bioavailability, is possible to reduce the total amount of cannabinoids that has to be metabolized by the enzymatic system of the liver.

A suitable dose can be determined by the clinician, dependent on the patient treated, e.g., weight, sex, and condition. Typically, cannabis active agents,. e.g., THC are titrated, i.e., the patient is first treated with a low initial does, and the dose is then increased to find the optimal dosing. Due to the improved bioavailability of the cannabis active agents with the pharmaceutical composition of the invention, as described herein, the initial dose can and should be significantly lower than with conventional drugs such as Sativex^{®}. Preferably, where at least one cannabis active agents is THC, the initial dose of THC administered is 0.025 -1 mg THC, preferably, 0.1 - 0.4 mg THC, optionally, 0.2-0.3 mg THC.

### Examples

### 1. Preparation of cannabis extract

1. Harvesting the fresh plant and pre-preparation: According to the desired profile of active agents, the plant is harvested at the optimal time point, and the florescences isolated.
2. Freezing: Cannabis flos is frozen by cryoconservation at the site of harvest. This blocks biological and enzymatic processes and prevents degradation and evaporation of the active agents.
3. Frozen sectioning: The still frozen florescences are broken up (preferably, the flowers are not broken up), at low temperature (10 to -20°C), and thus prepared for separation of the trichomes.
4. Ice water filtration: In this step, the trichomes of the florescences are separated from other plant material.
5. Collection of trichomes: The collected raw extract from the filtrate is a mirror image of the profile of active agents (in particular the THC:CBD ratio) of the plant from which it is derived, and it contains all active agents of the cannabis resin, but it also includes other undesired components.
6. Cavitation by ultrasound and winterisation in ethanol: For separation from undesired waxes, fat and lipids from the raw extract, the extract is dissolved in ethanol and then cooled. The cold allows for the separation of compounds through differences in their melting points and precipitation points. Thus, the fats and waxes precipitate at higher melting points, and can be separated off by filtration, centrifugation or decantation. A pure liquid oil extract remains.
7. Separation of volatile components from phytocannabinoids and other resin components: In this step, the easily volatile terpene-containing essential oils are distilled, and thus protected from degradation and evaporation upon the subsequent decarboxylation of the phytocannabinoids.
8. Decarboxylation: The inactive form of phytocannabinoids is transformed into the active form by the effect of heat.
9. Fusion of the oil extract and the terpene distillate. The two fractions, the terpenes from the distillate and the decarboxylated cannabinoids from the cannabis resin (oil extract) are again combined to obtain a full extract with a profile of active agents corresponding to the profile of the chemovar from which the plants were derived.
10. Standardization of the oil extracts
11. Encapsulation in the nanocarrier

Preferred methods comprise are:
Step 2: Preferably, directly after the harvest, and without drying, the plant material is frozen for the later preparation. Optimally, the florescences are trimmed, i.e., all larger stalks and leaves are removed before freezing, and larger florescences split. Freezing directly after harvest has the advantage that only a low amount of the highly volatile terpenes are lost. In the process of drying and maturation, typically, relevant amounts in particular of the monoterpenes are lost, and the profile of terpenes does not any more correspond to the profile at the time of harvest.

Further, we'd like to prevent that broken plant material from leaves and stalks adds impurities to the final product after the separation in ice water.

However, by freezing, the cell walls are broken, and there is a risk of co-isolating undesired substances such as chlorophyll. It is thus recommended to leave the trichomes as intact as possible, as their content otherwise acts as a solvent for lipophilic substances and to prevent the loss of pharmaceutical interesting ingredients to volatilisation.

Step 4: Ice water filtration of the trichomes of cannabis flos is carrier out without use of solvents. It is more of a mechanical step of separation than an extraction process, and it is based on the fact that the trichomes easily break from the plant material because of an increase in viscosity in the cold. The frozen plant material is added into an ice/water mixture (alternatively, ice/dry ice), and the mixture is agitated for some minutes (e.g., 3-10 min). It is recommended to carry out this agitation softly, and not to use electrical devices such as mixers, as these strongly increase the risk to contaminate the obtained extract with plant material.

The wax-containing trichomes break off the plant material due to this treatment and can be isolated e.g., with suitable sieves with decreasing mesh size (45 - 73 - 120 - 160 - 190 - 220 µm).

Step 5. A step for separating the trichome shell, which contains undesirable waxes and lipids, from the content of the trichomes follows. For this, the method of cavitation by high-energetic ultrasound, a subsequent wash, and cooling in ethanol (winterization) can be used. A mechanical process like cold pressing is an alternative. The raw resin obtained by this process has the same profile of active agents as the starting material.

The preferred process for obtaining the resin in substance corresponds to the traditional preparation of hashish, with the difference, that, because, preferably, there is no drying step after harvest, there is less contamination by dust from plant remains, and volatile substances are substantially maintained.

Due to the absence of solvents from the extraction processes, the process is less efficient, however, it is advantageous that the final product is not artificially changed, and mirrors the profile of active agents of the plant material used.

Step 7: Before the process of decarboxylation of the phytocannabinoids, the thermally labile terpenes are preferably isolated and protected from degradation or evaporation. The most widely used method for isolating volatile terpenes from plant material is steam distillation. This can be done in the context of decarboxylation, as the temperature required for decarboxylation can be slowly increased, wherein the thus distilled essential oils are captured. After this separation, the cannabinoids can be decarboxylated, and once this process is finished, mixed with the distilled terpenes to the final product.

Step 8, Decarboxylation: THC and CBD are the major bioactive components of cannabis. In cannabis plants, these substances are present in the form of biologically inactive carboxylic acids (THCA and CBDA). To transform these into their biologically active forms, a step of decarboxylation is required. THCA and CBDA naturally decarboxylate over time. The traditional way for increasing the speed of decarboxylation however is the use of heat over a defined time.

In this decarboxylation step, the functional carboxyl residue is eliminated from the cannabinoids by thermal treatment of the educt or the prepared extract, leading to formation of the more effective neutral form, e.g., THC and CBD.

Decarboxylation of cannabinoid acids is a function of time and temperature, so that with higher temperatures, a shorter time is required for total decarboxylation of a specified amount of cannabinoid acids. In the selection of suitable conditions for the decarboxylation, it must also be considered that the thermal degradation of desired pharmacologically active cannabinoids and other secondary plant metabolites should be minimized, in particular, the thermal degradation of THC to CBN (cannabinol). In laboratory analyses, optimal conditions for decarboxylation of CBDA to CBD from CBD-dominant chemovars of cannabis, as well as for decarboxylation of THCA to THC at temperatures, at which only a low amount of CBN is expected, were tested. It was shown that most efficient decarboxylation of CBDA to CBD occurs at 120-140°C for 30-60 min, e.g., 120°C for 60 min or 140°C for 30 min. Optimal conditions for decarboxylation of THCA to THC with minimal formation of CBN were 105-120°C for 30-120 min, e.g., 30 min at 120°C or 60-120 min at 105°C. Thus, the optimal conditions for decarboxylation depend on the cannabinoid to be decarboxylated. Optimally, a chemovar mainly producing CBD is decarboxylated for an hour at 120°C or 30 min at 140 °C. Optimally, a chemovar mainly producing THC is decarboxylated for 1-2 hours at 105°C or 1 hour at 120°C to minimize CBN formation.

Thus, for combined THC/CBD profiles, decarboxylation at 120°C for 60 min is expected to optimize yield in combination with minimal formation of CBN.

Preferably, in the context of the invention, the active agents are isolated from one variety of cannabis, and the profile of active agents largely mirrors the profile of agents (or their precursors) in the plant. For example, varieties comprising both THC and CBD in about an equal ratio (or e.g., at about 1:1, about 1:2 or about 1:3) can be used. However, for a ratio of THC:CBD that can be freely chosen, blending of two cannabis varieties can be performed, one THC dominant and one CBD dominant variety. The use of two homozygous plants leads to a more reliable result in this than growing heterozygous plants that can produce both THC and CBD, as, depending on environmental factors, such plants may have a high variability in the content of their active agents. However, the environment under which the plants are grown can be largely standardized, which can reduce variability.

Step 9: After these steps, the two fractions, terpenes from the distillate and the decarboxylated cannabinoids in the cannabis resin are preferably again combined to provide a total extract with a profile of active agents corresponding to that of the chemovar. This oily and purified extract can be further processed, and e.g., encapsulated in a nanocarrier as described herein.

### 2. Preparation of nanocarrier

a) Nanocarrier can be prepared as described in WO 2015/172769 A2. These nanocarriers are particularly suitable for transdermal or transmucosal administration.
b) Nanocarriers particularly suitable for intravenous administration, wherein the effect of an associated drug (e.g., THC) in the CNS is reduced, are, e.g., CMS-amide C18-mPEG350, can be prepared as described in detail in EP 1796649. This carrier contains free positively charged amino groups. Further suitable nanocarriers are CMS Ester C15 mPEG350 type. This nanocarrier can be prepared as described in Example 1 of WO 2017/097291 A1.

### 3. Preparation of a composition according to the invention comprising nanocarriers and cannabis active agents without a covalent link

CMS Nanocarrier of the type hPG (Mn 10k) (octadecanedioic mPEG350 ester) amide (degree of functionalization between 55 and 75%) and a cannabis extract of a chemovar comprising THC and CBD at an approximately equal ratio, and prepared as described in Example 1, in a ratio of nanocarrier : cannabis active agent of 5:1 (w/w) are dissolved in 0.9% NaCl. The samples are stirred at 1200 rpm for 22 h. Subsequently, a sterile filtration (200 nm RC) is made.

### 4. Mucosal administration of a composition of the invention comprising nanocarriers and cannabis active agents without a covalent link

The composition may be a spray for transmucosal administration, in particular, for oromucosal administration to a patient, e.g., for buccal and/or sublingual administration. For example, in a composition of the invention comprising 1mg/mL of THC, 100 µL (e.g., one spray) of the composition comprises 100 µg THC. The THC: CBD ratio may e.g., be 1:1, 1:2 or 1:3. A dose escalation scheme for starting therapy, based on a bioavailability of 75% could be, e.g.,:

| | |
|---|---|
| Day 1. | 0 µl in the morning / 100-300 µl in the evening |
| Day 2. | 0 µl in the morning / 200-400 µl in the evening |
| Day 3. | 0 µl in the morning / 300-500 µl in the evening |
| Day 4. | 100-300 µl in the morning / 400- 600 µl in the evening |
| Day 5. | 100-300 µl in the morning / 500-700 µl in the evening |
| Day 6. | 100-300 µl in the morning / 600-800 µl in the evening |
| Day 7. | 200-400 µl in the morning / 700-900 µl in the evening |
| Day 8. | 300-500 µl in the morning / 800-1000 µl in the evening |
| Day 9. | 400-600 µl in the morning / 900-1100 µl in the evening |
| Day 10. | 500 -700 µl in the morning /1000-1200 µl in the evening |

The patient starts with the scheme and stops escalating the dose when his or her needs are met, e.g., when the pain is reduced to a manageable or desired level.

The scheme can be adapted for different bioavailabilities or concentrations, or depending on the need of the patient.

## Claims

1. A composition or kit comprising
a) a hydrophilic nanocarrier comprising a hyperbranched dendritic polymer selected from the group comprising a core-multishell-nanocarrier and a core-shell nanocarrier, and
b) a plurality of cannabis active agents, preferably, a cannabis extract,
wherein, preferably, a) and b) are in one composition.

2. The composition or kit of claim 1, wherein the cannabis active agent is THC (tetrahydrocannabinol), CBD (cannabidiol), THCA (tetrahydrocannabinolic acid), CBN (Cannabinol), CBDA (cannabidiolic acid), CBG (cannabigerol), CBC (cannabichromene),, THCV (tetrahydrocannabivarin), THCP (tetrahydrocannabiphorol) and CBDV (cannabidivarin) or a synthetic cannabinoid selected from the group comprising Nabilone (synthetic THC), biosynthetic cannabigerol, biosynthetic cannabidiol, and synthetic THCV, or a terpene selected from the group comprising β-myrcene, β-caryophyllene, α-humulene, α-pinene, limonene, linalool, bisabolol and (E)-β-farnesene.

3. The composition or kit of any of the preceding claims, wherein one of the cannabis active agents is THC, preferably, Δ⁹-THC.

4. The composition or kit of any of the preceding claims, wherein one of the cannabis active agents is CBD.

5. The composition or kit of any of the preceding claims, comprising b) THC and CBD, optionally, in a defined ratio.

6. The composition of any of the preceding claims, wherein the concentration of cannabis active agents is stable for at least 6 months, preferably, for at least 12 months, more preferably, for at least 24 months.

7. The composition of any of the preceding claims, wherein the ratio of THC to CBD is stable for at least 6 months, preferably, for at least 12 months, more preferably, for at least 24 months.

8. The composition of any of the preceding claims, wherein at least one active agent, preferably, CBD, is non-covalently associated with the nanocarrier, wherein, optionally, all active agents are non-covalently associated with the nanocarrier.

9. The composition of any of the preceding claims, wherein at least one active agent, preferably, THC, is covalently associated with the nanocarrier,
wherein, optionally, a plurality of cannabis active agents is covalently associated with the nanocarrier.

10. The composition or kit of any of the preceding claims, wherein the nanocarrier is a hyperbranched dendritic polyglycerol polymer or a hyperbranched dendritic polylactate polymer or a hyperbranched dendritic polyglycerol-polylactate polymer, preferably, a hyperbranched dendritic polyglycerol polymer.

11. The composition or kit of any of the preceding claims, wherein the nanocarrier consists of a dendritic core and at least two shells, wherein an inner shell is associated with the dendritic core via a linker, and an outer shell is associated with the inner shell with a linker,
wherein the nanocarrier optionally is a negatively or positively charged nanocarrier, preferably, a negatively charged nanocarrier.

12. The composition or kit of any of the preceding claims, wherein the nanocarrier has a molecular weight of 100 g/mol-990 g/mol, preferably, 200-800 g/mol.

13. The composition or kit of any of the preceding claims, wherein the nanocarrier has a molecular weight of 1,000 g/mol-100,000 g/mol, preferably, 3,000-10,000 g/mol.

14. A pharmaceutical composition or kit comprising the composition or kit of any of the preceding claims, optionally further comprising at least one excipient selected from the group comprising a mucoadhesive agent, a flavouring agent and a palatability enhancer.

15. The pharmaceutical composition or kit of claim 14, for use in treating a condition selected from the group comprising pain, e.g., chronic pain or acute pain, neuropathic pain, inflammation-induced pain, pain associated with osteoarthritis, pain associated with cancer, seizures, e.g., seizures associated with Dravet syndrome, seizures associated with Lennox-Gastaut syndrome, or tuberous sclerosis complex or epilepsy, multiple sclerosis, spasticity, overactive bladder, increased intraocular pressure, emesis, insomnia, cancer, anorexia, cachexia, or for muscle relaxation, reducing gastric motility, and/or increasing appetite.

16. The pharmaceutical composition or kit for use of any of claims 14 or 15, wherein the composition is administered transmucosally, transdermally or intravenously, preferably, by oro-mucosal application, wherein, in case of a kit, the components of the kit are preferably mixed before administration.

17. The pharmaceutical composition of kit for use of any of claims 14-16, wherein at least one cannabis active agents is THC, and wherein the initial dose of THC administered is 0,025 mg -1 mg THC, preferably, 0.1 - 0.4 mg THC, optionally, 0.2-0.3 mg THC.

18. The pharmaceutical composition or kit for use of any of claims 14-17, wherein the composition is or the components of the kit are administered in combination with an analgesic selected from the group comprising an opioid, an NSAID, an anti-inflammatory agent, and antiepileptic agent and an antidementia agent.
